# EUROPÄISCHE PATENTANMELDUNG

(11) **EP 0 815 824 A2**
(43) Veröffentlichungstag der Anmeldung: **07.01.1998**
(21) Anmeldenummer: 97810406.5
(22) Anmeldetag: 25.06.1997
(51) Int. Cl.: A61H 39/00, A61N 5/06

(54) **Hülsenaufsatz auf Soft-Laser-Handgerät mit auswechselbaren Düsen**

(30) Priorität: 05.07.1996 CH 1680/96
(71) Anmelder: Sagarra, Louis, 8405 Winterthur (CH)
(72) Erfinder: Sagarra, Louis, 8405 Winterthur (CH)

(57) **Zusammenfassung**

Vorrichtung die unlösbar mit einem Laserlicht abstrahlenden Handgerät verbunden und zur Aufnahme verschiedener Düsenaufsätze mittels Gewinde ausgebildet ist. Die bisher verwendeten Soft-Laser weisen mehrheitlich eine zu grosse AustrittsÖffnung aus um eine gezielte Akupunkturtherapie zu ermöglichen. Dies wird hiermit vermieden, da mehrheitlich die Akupunktur-Reizpunkte, zur Hauptsache in der Ohrengegend sehr eng beieinander liegen.

## Beschreibung

Die vorliegende Erfindung betrifft eine Bestrahlungsvorrichtung zur gezielten Akupunktur-Therapie einer Person mittels eines Soft-Laser Handstückes welches unlösbar mit einer Hülse verbunden ist, die zur Aufnahme von Düsen mittels Gewinde ausgebildet wurde. In diese Hülse können nun verschiedene Düsen eingeschraubt werden, die den roten Laserstrahl beim Austritt aus dem Handstück von ca. 10 mm im Durchmesser, je nach Austrittsöffnung der eingeschraubten Düse, auf 2,5 mm, 2 mm oder 1,5 mm im Durchmesser reduzieren.
In den letzten Jahren wurde vermehrt auf die Naturheilkräfte und Naturheilmethoden zurückgegriffen um den Körper zu regenerieren und zu entgiften. Vor allem wurde auf die fernöstliche Heilmethode der Akupunktur mittels Nadeln zurückgeriffen, die aber aufwendig und zeitraubend ist. Ende der achtziger Jahre wurden erstmals Soft-Laser in der Akupunkturtherapie eingesetzt, welche sich auch bewährt haben. Nur besassen diese den Nachteil, dass bei allen Soft-Laser Handstücken die Austrittsöffnung des roten Laserlichtes viel zu gross Ausgebildet war. Dies wirkte sich vor allem bei Akupunktur-Therapien bei welchen die Reizzonen an den Ohren bzw. an den Füssen lagen, die Reizpunkte liegen hier sehr nahe beieinander, nicht unbedingt negativ aber auch nicht unbedingt vorteilhaft aus, da mit einem Lichtstrahl von ca. 10 mm Durchmesser meisst mehrere Reizpunkte gleichzeitig aktiviert wurden, d.h. es konnte keine gezielte Akupunktur-Therapie durchgeführt werden.
Eine gezielte Anwendung des roten Soft-Laserlichtes auf der Haut kann eine gesunde Körperreaktion bewirken und die Gesundheit einer Person verbessern.
Weiter kann diese sichtbare Laser-Lichtquelle dazu beitragen, die Gesundung von Personen zu verbessern. Der Laserstrahl hat eine stimulierende Wirkung auf Wundheilung und Gewebe. Er bewirkt eine Vermehrung kollagener Fasern und Vesikel (Vesikel enthalten vermutlich "bioaktive" Substanzen, die die Heilung auch in nichtbestrahlten Gebieten katalisieren), er bewirkt eine Verstärkte Aderneubildung (Neovaskularisierung), eine erhöhte Enzymaktivität (Succinyldehydrogenase), eine Steigerung der Zugfestigkeit von Wunden, eine Vermehrung der Zellen in der Teilungsphase, eine Stimulierung der Proteinsynthese und eine Verbesserung der Zellatmung (u. a. Stimulierung der Zytochromoxydase).

Ziel der Erfindung ist es, den oben erwähnten Nachteil zu beheben.

Die Erfingungsgemässe Soft-Laser-Bestrahlungsvorrichtung ist durch die Merkmale des Patentanspruchs 1 gekennzeichnet.

Nachfolgend wird der Erfindungsgegenstand anhand der Zeichnungen beispielsweise näher erläutert.

### Es zeigt:

Figur 1 eine vereinfachte Ansicht zur Erklärung einer Ausführung eines bekannten Soft-Laser-Behandlungs-Handstückes zur Therapie-Behandlung.

Figur 2 Querschnitt zur Erklärung einer Ausführung des gemäss der vorliegenden Erfindung ausgebildeten Laserlicht abstrahlende Gerätes zur gezielten Akupunktur-Therapie (Hülse).

Figur 3 Querschnitt zur Erklärung einer Ausführung des gemäss der vorliegenden Erfindung ausgebildeten Laserlicht abstrahlende Gerätes zur gezielten Akupunktur-Therapie (Düse).

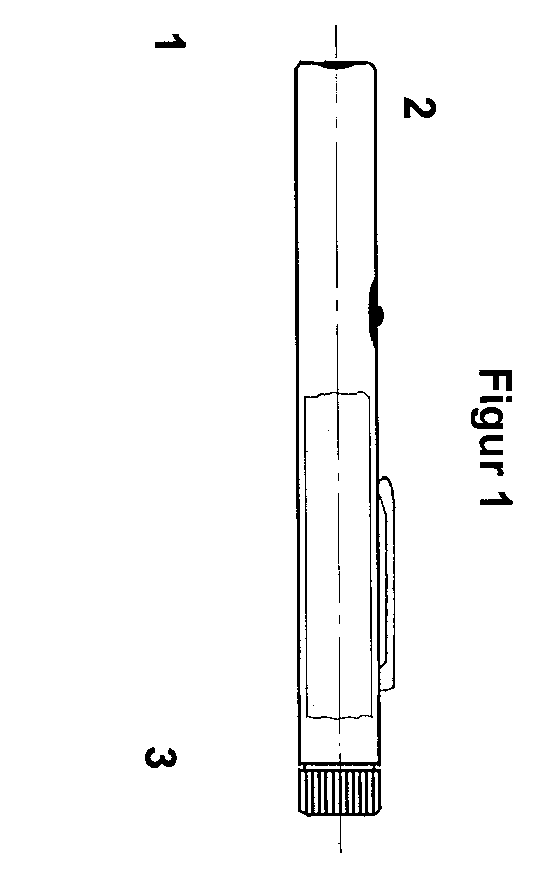

Figur 1 zeigt vereinfacht eine Illustration einer Ausführung eines Soft-Laser-Handgerätes zur Akupunktur-Therapie, die gegenwärtig durch den Anmelder vertrieben wird. Dieses Handstück (Gehäuse) enhält auf der einen Seite (1) eine Austrittsöffnung für den roten Laserlichtstrahl, auf der Oberseite (2) einen Druckknopf um den Laserlichtstrahl einzuschalten, und auf der anderen Seite einen Schraubverschluss (3) hinter dem sich das Batterie-Einschubfach befindet.

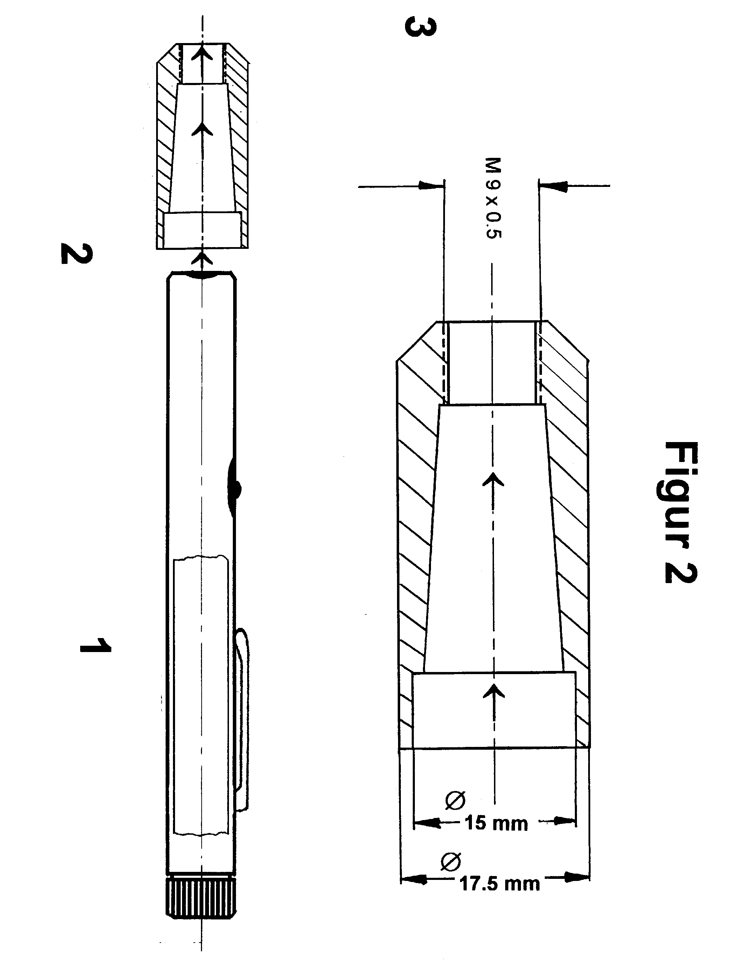

Figur 2 zeigt im Querschnitt die Hülse, die mit dem unter Figur 1 beschriebenen Soft-Laser-Handstück fest verbunden wird, welches gemäss der vorliegenden Erfindung ausgebildet ist. In der Figur 2 bezeichnet die Bezugsziffer 1 das bestehende Laser-Lichtstrahlen-Handstück. Die Bezugsziffer 2 die Austrittsöffnung des roten Laserlichtes. Die Bezugsziffer 3 die Austrittsöffnung des Soft-Laserlichtes an der Hülse mit Angabe der Gewindegrösse.

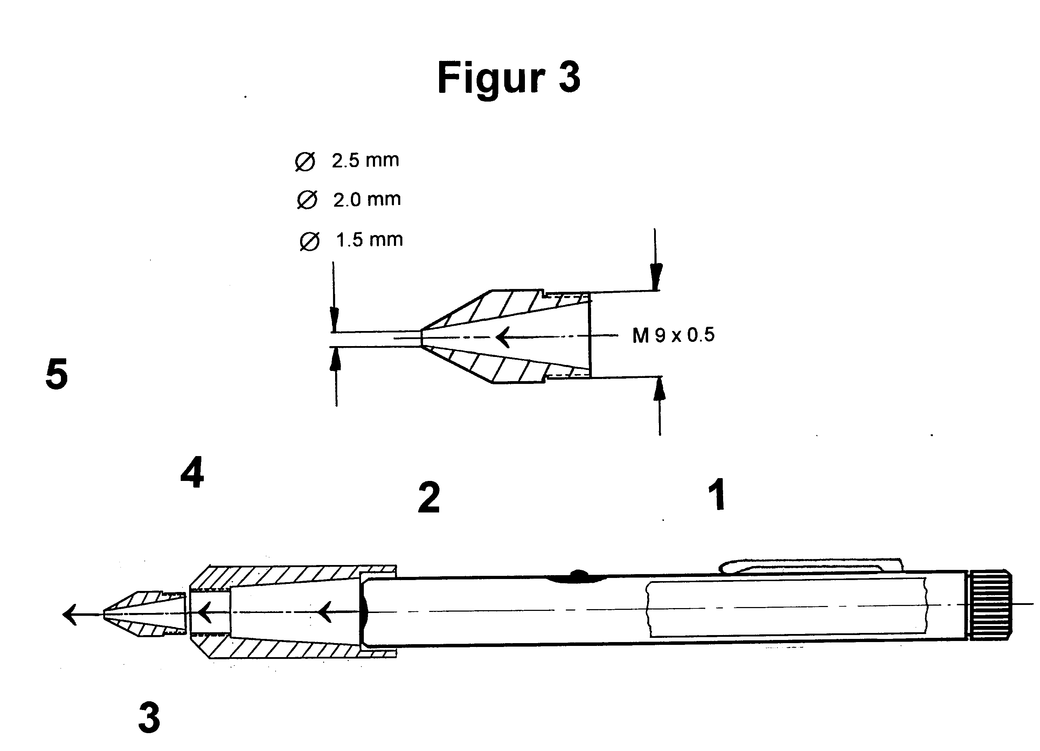

Figur 3 zeigt im Querschnitt die Düse, die mit der unter Figur 2 beschriebenen Hülse durch Gewindeverschraubung lösbar verbunden wird, welches gemäss der vorliegenden Erfindung ausgebildet ist. In der Figur 3 bezeichnet die Bezugsziffer 1 das bestehende Laser-Lichtstrahlen-Handstück. Die Bezugsziffer 2 die Austrittsöffnung des roten Laserlichtes. Die Bezugsziffer 3 die Austrittsöffnung des Soft-Laserlichtes an der Hülse mit Angabe der Gewindegrösse. Die Bezugsziffer 4 die Eintrittsöffnung an der Düse mit Angabe der Gewindegrösse. Die Bezugsziffer 5 die Austrittsöffnung an der Düse mit Angabe der verschiedenen Austrittsöffnungen der verschiedenen Düsen.

## Patentansprüche

1. Vorrichtung (Hülse) die unlösbar mit einem Laserlicht abstrahlenden Handgerät verbunden wird und zur Aufnahme verschiedener Düsenaufsätze mittels Gewinde ausgebildet ist.
Düsen nach Anspruch 1, die mittels Gewinde in die unlösbar mit einem Soft-Laser verbundene Hülse geschraubt werden, um eine verkleinerung des Laserlichtaustrittes zu erreichen.
